(19) Europäisches Patentamt
European Patent Office
Office européen des brevets

(11) **EP 4 231 313 A1**

(12) **EUROPEAN PATENT APPLICATION**

(43) Date of publication:
**23.08.2023   Bulletin 2023/34**

(21) Application number: **23156600.1**

(22) Date of filing: **14.02.2023**

(51) International Patent Classification (IPC):
*G16H 50/20* (2018.01)    *G06N 7/01* (2023.01)
*G06N 20/00* (2019.01)    *G16H 30/40* (2018.01)
*G16H 40/40* (2018.01)    *G16H 50/30* (2018.01)
*G16H 50/70* (2018.01)

(52) Cooperative Patent Classification (CPC):
**G16H 50/20; G06N 7/01; G06N 20/00; G16H 30/40;
G16H 40/40; G16H 50/30; G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.02.2022   US 202217677970**

(71) Applicant: **Optellum Limited
Oxford, Oxfordshire OX1 1BY (GB)**

(72) Inventors:
• **Waterfield-Price, Noah
Oxford, OX1 1BY (GB)**
• **Dowson, Nicholas
Oxford, OX1 1BY (GB)**

(74) Representative: **Optimus Patents Limited
Peak Hill House
Steventon
Basingstoke, Hampshire RG25 3AZ (GB)**

(54) **CADX DEVICE AND A METHOD OF CALIBRATION OF THE DEVICE**

(57)    A CADx system and method for analysing a medical image from a local population for a disease risk score and determining if the system is calibrated is described. The CADx system comprising :an input circuit for receiving at least one medical image and producing CADx derived data based on the received image; a machine learning model for determining a disease risk score for the medical image; a calibration auditor circuit for determining a calibration state of the CADx system by: receiving the CADx derived data and comparing the CADx derived data to training derived data, where the training derived data comprises one of more of: training data for the machine learning model for determining the disease risk score, and data on population factors associated with the training data; and an output circuit for outputting a disease risk score for the medical image and an indication of the calibration state.

**FIG. 1a**

**Description**

**Field of Invention**

This invention relates to the field of Computer Aided Diagnosis (CADx) systems and methods for assisting determination of medical images, used to support clinicians in healthcare. In particular, the field relates to risk Computer Aided Diagnosis systems, determination of whether a CADx system is calibrated, the calibration of the CADx system, where the CADx system assists the reading and reporting of medical images by radiologists and the interpretation of the radiologist's report by the physician responsible for patient care.

**Background of Invention**

In the field of medical imaging, a variety of technologies can be used to investigate biological processes and anatomy. The following examples are types of scan that may be used to provide medical images: X-Ray; Computed Tomography (CT); Ultrasound (US); Magnetic Resonance Imaging (MRI); Single Photon Emission Tomography (SPECT); and Positron Emission Tomography (PET). Each type of scan is referred to as an "imaging modality".

Typically, a scan provides a "dataset". The dataset comprises digital information about the value of a variable at each of a plurality of spatial locations in either a two-dimensional or (more typically) a three-dimensional space. As a specific example, a CT scan may provide images of the chest of a patient. Such a CT scan might, as a more specific example, show lung nodules in the chest.

Computer Aided Detection (CADe) devices serve to assist its users (e.g. typically clinicians) in assessing the medical images. CADe devices need to provide a clinician with standardised, objective and repeatable information. The information typically relates to particular anatomical regions, including both normal tissue and lesions, within a person. CADe devices may be used as a so-called 'Second Reader' system. Second Reader Systems are based on an approach whereby a radiologist first looks at an image resulting from a scan, for example a mammogram. The radiologist will then, based on training and experience, identify areas of the scan where the radiologist considers that there may need to be a further investigation, for example a biopsy. However, the radiologist can then consider the CADe findings. Those findings might involve a display to highlight any additional suspicious regions on the mammogram. The radiologist will then, based on training and experience, look at those further areas of the scan. The CADe device is thereby performing a second look at the scan. The results of the second look at the scan may be that the radiologist will be directed to areas of the scan that he/she had overlooked. In this way, CADe devices are designed to reduce 'false negatives', which are also termed 'missed findings'. Thus CADe devices perform a support role to clinicians.

Computer Aided Diagnosis (CADx) devices are a related technology to CADe. CADx devices attempt to solve a different problem and relate generally to risk assessment. Instead of focussing on potentially missed findings as in CADe, they try to assist the user to classify findings correctly, either as malignant or benign in the case of potentially cancerous lesions. They rely on the user to identify abnormalities, but then typically provide a score that is indicative of the risk of malignancy. There are many examples of such CADx devices proposed within the academic literature. However, few systems are available commercially, and hence used in clinical practice. This discrepancy is indicative of the difficulties in deploying practical systems with the known approaches. The output of known CADx devices is typically some kind of score. That score indicates the risk or likelihood of disease, or its absence. An example of a commercial CADx device is the 'Transpara™' product from 'Screenpoint™'. There are many non-clinical CADx devices in the academic literature.

State-of-the-art CADx devices are built around *machine learning models.* These models are generic algorithms with "learnable" parameters which are fitted using training data such that the model can be used to make predictions on previously unseen data. For example, a machine learning model built to predict whether a lung nodule on a CT image is malignant, can be trained using a collection of datasets of CT images containing lung nodules that have an associated label, for example nodule malignancy may be indicated by a one, and nodule benignity may be indicated by a zero. Such models are used to assist the clinician in estimating the risk that a lung nodule they found in their practice is malignant. Machine learning models operate by applying a series of mathematical operations defined within a *feature encoder* to any *input data* provided to the input of the model to generate a collection of numbers called *features*. The features describe the extent to which a particular pattern or its antithesis is present within the input data. Subsequently, the features have another set of mathematical operations, defined within an *output module,* applied to them to generate a *raw disease risk score.* The raw disease risk score may be difficult to interpret, so it is typically supplied to a *score mapper,* which performs a series of mathematical operations on the raw disease risk score to generate a *disease risk score* that indicates the likelihood of disease and is more readily interpreted by clinicians. The mathematical operations in the feature encoder, the output module and the score mapper are controlled by a collection of scalar floating-point numbers collectively referred to as *machine learning model parameters*. For the machine learning model to serve a useful purpose, the parameters need to be generated using a process known as model *training.* The data used to train the machine learning model parameters is known as *training data* and comprises of a collection of *input training data* where each datum is associated with a *label* defining whether the disease is present or not. Once the machine learning model parameters have been trained, the model can be used to make a prediction on previously unseen input data to generate disease risk scores in a process known as *inference.* The disease risk score output by the *inference* process in the case of a machine learning-based CADx device could for instance indicate the likelihood of malignancy for a lung nodule.

A general assumption when using machine learning models is that the training data will be representative of the data input to the model at inference time, i.e. once the CADx model is deployed and in use by medical personnel. For a system deployed at a clinic, parameters in the score mapper may need to be refined or *calibrated* subsequent to model training to reflect the prevalence of the disease within the population of patients served by the clinic and also compensate for other *local population factors* that affect disease prevalence such as the distribution of age, race and sex. All systems whether calibrated or not, suffer from a certain level of error, because the information used predict the disease is limited and itself prone to error. In cases where the system over-estimates the likelihood of malignancy the result is a "false positive" which may result in the patient being referred for an unnecessary biopsy. Alternatively, the model may also under-estimate the likelihood of malignancy, resulting in a "false negative" finding, which may result in a delay in the diagnosis of cancer and a worse outcome for the patient. Hence uncalibrated or miscalibrated CADx devices suffer from increased rates of error, which can have serious consequences for patients.

Consider the specific example of a system for predicting whether lung nodules are malignant or benign. The system may have been trained using a population of patients from Europe and outputs an integer for the disease risk score ranging from 1 to 10. The system may have been calibrated so that 10% of patients receiving a score of 1 actually have cancer, 20% of patients receiving a score of 2 actually have cancer, and so on. The system is deployed in a clinic located in the South Eastern United States, where the rates of histoplasmosis are high relative to Europe, resulting in an increased frequency of benign lesions that resemble malignant nodules. As a result, a higher number of patients receive biopsies than is necessary. After some time, the clinic recognises that the rate of over-diagnosis has increased and compensates by only referring patients for biopsies if they receive a very high score. However, this means that a number of patients with early-stage lung cancer who have a low score do not receive a biopsy resulting in a delayed diagnosis. In some cases, the delay is so long that the cancer becomes incurable leading to the untimely death of some patients of the clinic.

The need to calibrate of the raw disease risk scores output by CADx models has long been recognised and many approaches to calibrate models have been proposed such as calibration for example Platt scaling [1] and Isotonic regression [2].

Even though the consequences of an uncalibrated system can be serious, in practice systems are seldom calibrated to their local population for several reasons. CADx devices can become uncalibrated over time. CADx systems are typically trained and calibrated using data from patient populations different to those found at individual sites. For some racial groups and regions certain conditions are more common, for instance African American's are more likely to suffer from sarcoidosis [3]. The volume of data required for training is typically very large and requires examples of patients both with and without disease. For comparatively rare disease like cancer, the volume of disease-positive data required for good results can exceed the total population of a particular geographic region, precluding the possibility of training a model specific to that region. The population resident in an area can change seasonally, for instance in regions where the main economic activity is farming and have an influx of migrant farm workers at harvest time. New fast-spreading diseases, such as COVID-19, can cause an increase in the presence of artifacts that can create a bias in the score output by a CADx system. Bias and hence miscalibration can also arise when sites can start using a new imaging protocol or purchase a new CT scanner.

Even more importantly, the inventors have recognised that it may be impossible for those using a CADx device to even identify that it is uncalibrated. As a result, patients can receive poor treatment in the long term. The difficulty in identifying when a CADx device is uncalibrated arises because the effects of being uncalibrated are subtle and require measuring treatment efficacy for a large number of patients over a long period of time by someone skilled in statistical techniques. Few facilities have the resources available for such an effort and institutions may be reluctant to record or report cases of a CADx device being uncalibrated. There is also a previously unrecognised need to enable CADx devices to work out when enough data has been accumulated to perform a recalibration.

Even when a CADx device is identified to be uncalibrated, existing approaches to recalibrate complex models such as convolutional neural networks do not utilise such models' intrinsic ability to work out what features are associated with population factors that affect the probability of disease and hence also affect the CADx device's calibration.

This invention proposes an approach to address all the above problems, namely:

* identifying when CADx device needs to be recalibrated
* provide an approach to recalibrate complex models
* checking if sufficient data is available to perform each of these steps.

[1] Platt, J "Probabilistic outputs for support vector machines and comparisons to regularized likelihood methods". Advances in Large Margin Classifiers. 1999; 10 (3): 61-74.

[2] Niculescu-Mizil A and Caruana R. "Predicting good probabilities with supervised learning," ICML '05: Proceedings of the 22nd international conference on Machine learning. August 2005; 625-632, https://doi.org/10.1145/1102351.1102430.

[3] Mirsaeidi M; et al. "Racial Difference in Sarcoidosis Mortality in the United States," Chest. 2015 Feb; 147(2): 438-449.

## Summary of the Invention

Accordingly, the invention seeks to mitigate, alleviate or eliminate one or more of the abovementioned disadvantages singly or in any combination.

According to the invention there is provided a CADx system for analysing at least one input medical image from a specified local population for a disease risk score and determining if the system is calibrated for the local population comprising: an input circuit for receiving at least one input medical image and producing CADx derived data based on the received input image; a machine learning model for determining a disease risk score for the input medical image; a calibration auditor circuit for determining a calibration state of the CADx system by: receiving the CADx derived data and comparing the CADx derived data to training derived data, where the training derived data comprises one of more of: training data for the machine learning model for determining the disease risk score, and data on local population factors associated with the training data; and an output circuit for outputting a disease risk score for the input medical image and an indication of the calibration state for the CADx system.

Preferably, the calibration auditor circuit further comprises a safety lock that prevents outputting of the indication of the calibration state when there is insufficient CADx derived data to determine the calibration state.

Further preferably, the calibration auditor circuit further comprises a calibrator to recalibrate the CADx system when the calibration state is determined to be uncalibrated for the specified local population.

In an embodiment of the invention, the calibration auditor circuit further comprises a second safety lock for the calibrator that prevents recalibration of the CADx system when there is insufficient CADx derived data for the recalibration of the CADx system.

Preferably, the calibration auditor circuit further comprises a threshold for calibration determination, wherein, when the difference obtained by comparing the CADx derived data and the training derived data, exceeds the threshold, the CADx system indicates that it is uncalibrated.

In a preferred embodiment of the invention, the CADx derived data includes a normalised distribution of the disease risk score for the input data, and the training derived data includes a normalised distribution for a disease risk score in the training data, and these distributions are used for the comparison.

Further preferably, the comparison of the CADx derived data with the training derived data is performed using a statistical divergence model, where the statistical divergence model is one of more of: a Kullbach-Liebler Divergence

$$\sum_{y' \epsilon Y} \rho(y'; \alpha) \log \frac{\rho(y'; \alpha)}{\rho_{y'}^p(y')}$$

(KLD) where $D_{KL} =$ ⎯ , where p(y'; α) is calculated from the training data dt, and $p^p_{y'}$ is calculated from the CADx derived data, or Kolmogorov-Smirnov statistic, the Anderson-Darling test, or Kuiper's test.

Preferably, the threshold is a predetermined threshold that is set either during training of the machine learning model or at the time of installation of the CADx system.

Further preferably, the CADx system is recalibrated, after the determination that the system is uncalibrated.

In an embodiment of the invention, the input medical image is one of: a CT image, an MRI image, a PET image, an X-ray image, an ultrasound image or a SPECT image.

Further preferably, the input further comprises one or more of: biomarkers for the patient or clinical parameters for the patient; wherein the biomarkers and clinical parameters comprise at least one of: patient age, patient sex, results of blood tests, results of lung function tests.

In an embodiment of the invention, the indication of the calibration state is an audio or visual output giving an indication that the CADx system is calibrated or uncalibrated for the local population.

According to the invention there is also provided a method of determining that a CADx system is uncalibrated for a particular local population, comprising the steps of: providing an input to the CADx system, comprising at least one medical image from the particular local population, to a machine learning model to provide at least one CADx derived data; providing the at least one CADx derived data from the machine learning model to a calibration auditor, where the calibration auditor compares the CADx derived data to calibration training data, wherein the calibration training data comprises one of more of: training data for the machine learning model, data on local population factors associated with the training data; and when the difference between the CADx derived data and the calibration training data exceeds a predetermined threshold, the CADx system is determined to be uncalibrated for the particular local population.

Preferably, the CADx derived data is one or more of data derived from the CADx input, intermediate data from the machine learning model or outputs from the CADx system.

In an embodiment of the invention, the CADx-derived data includes the local population factors; wherein the local population factors comprise one of more of age distribution, sex distribution, race or ethnicity distribution for the local population, seasonal variations in weather for the area of the local population; parameters related to the image scanner for the at least one medical image input.

Preferably, when the CADx system is determined to be uncalibrated, the CADx system is recalibrated for the local population by the following steps: activating a calibrator within the calibration auditor; using the activated calibrator to update the machine learning model;

Preferably, if the CADx system determines that there is not sufficient data in the calibration auditor database, a safety lock prevents the CADx system from being recalibrated.

In an embodiment of the invention, the machine learning model comprises at least an output module and a score mapper, and the calibrator updates the machine learning model by retraining at least one of the output module and score mapper using at least one of features $v_c$ and diagnoses $z_c$ from the CADx-derived data and at least one of features vt and diagnoses zt from the training-derived data.

Preferably, the calibration auditor further comprises a threshold for recalibration, wherein, when a comparison of the the CADx derived data with the training derived data, exceeds the threshold, the CADx system is recalibrated .

Further preferably, the comparison of the CADx derived data and the training derived data is done with a statistical divergence model that is one of more of: a Kullbach-Liebler Divergence (KLD) where

$$D_{KL} = \sum_{y' \epsilon Y} \rho(y'; \alpha) \log \frac{\rho(y'; \alpha)}{\rho^p_{y'}(y')}$$

, where p(y'; $\alpha$) is calculated from the training data dt, and $p^p_{y'}$ is calculated from the CADx derived data, or Kolmogorov-Smirnov statistic, the Anderson-Darling test, or Kuiper's test.

## Brief description of the drawings

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

Figure 1(a) illustrates a CADx system according to an embodiment of the invention;

Figure 1(b) 1a illustrates a CADx device **(120)** according to a further embodiment of the invention;

Figure 2 show a more detailed example implementation of a calibration auditor of Figure 1;

Figure 3 shows an alternative embodiment of a CADx system of the invention;

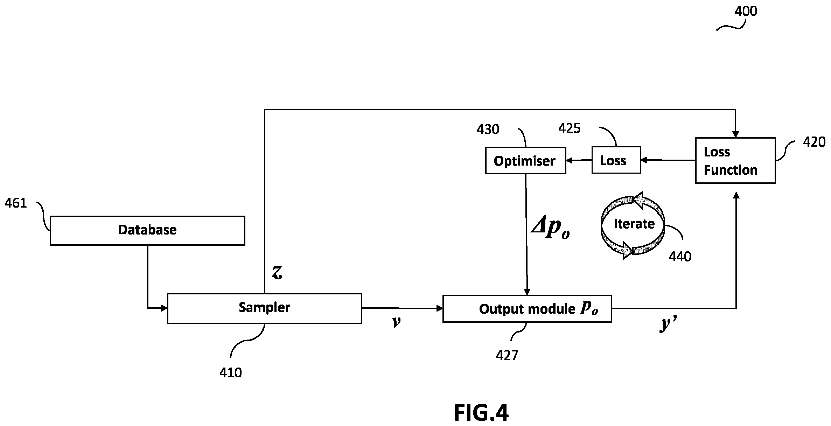

Figure 4 shows an example of the invention for mapping between different machine learning parameters;

## Detailed Description

### Overview of the invention

This invention addresses two previously unrecognised needs for CADx devices, namely: an internal system to assess if the CADx device is uncalibrated to a local population, and, given the device is uncalibrated, to optionally recalibrate it to a local population. For the latter task, the invention proposes a device that is applicable to recalibrating complex machine learning models, such as CNNs (convolutional neural networks). The invention is also able to identify when sufficient data from is available to perform each of these tasks..

The technical step made by the invention is to include a *calibration auditor circuit,* which is responsible for generating a *calibration assessment* and, optionally, recalibrating the CADx device. The calibration auditor analyses *CADx-derived data* and compares it to *training-derived data,* to determine if the CADx device is uncalibrated, and, optionally, recalibrate the CADx device. From the differences between the CADx-derived data and the training-derived data, as measured by a *calibration model,* the calibration auditor can identify when the CADx device has become uncalibrated and alert the user by providing a calibration assessment, which is output by the CADx device. Additionally, given relevant differences between the CADx-derived data and training-derived data, the calibration auditor may use a *calibrator* to minimise these differences and recalibrate the CADx device.

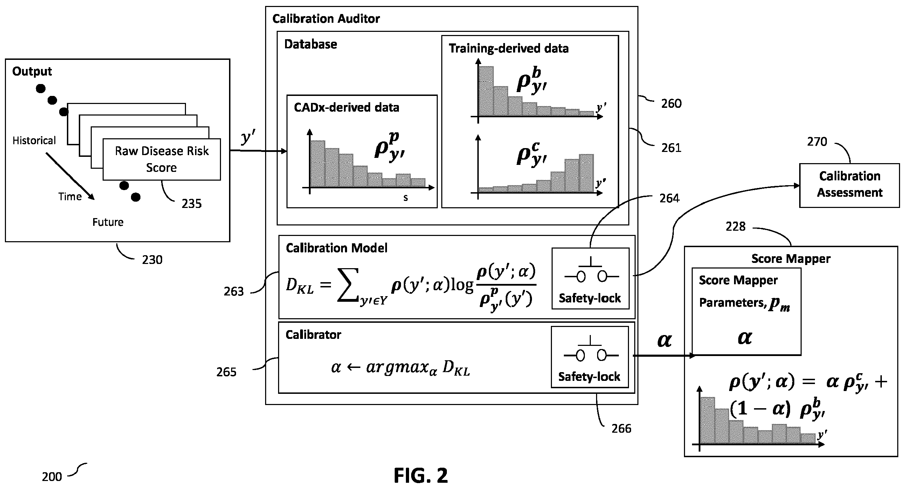

Due to the statistical nature of the operations performed by the calibration auditor, a minimum amount of CADx-derived data and training-derived data is required for the calibration assessment to be statistically meaningful and for the calibrator to be able to recalibrate the model. Different embodiments of the calibration auditor utilise operations that vary in their data requirements. In a clinical setting, the CADx-derived data will typically be collected over a period of time until enough data has been collected. This time period will depend upon both the embodiment of the calibration auditor and the rate at which data is collected, for a given site. There is no upper bound on the amount of data that may be used as input to the calibration auditor. For example, if a calibration auditor performs a simple analysis with relatively low data requirements, such as that illustrated in FIG 2 (described in the following section "An implementation of the calibration auditor"), the minimum amount of data could be as low as 100 datasets. In which case, a clinic could collect this amount of data of a several weeks or months. However, given that using more data will result in more significantly meaningful results, it may be preferable to use more datasets than the minimum required, which would have been collected over a much longer period, which could be several years or even decades.

To account for these data requirements, in some embodiments of the invention, the calibration model may contain a *safety lock* to ensure that a calibration assessment, or an indication of the calibration state is not provided if there is not sufficient data to do so. Similarly, in a preferred embodiment of the invention the calibrator may also contain a safety lock to ensure recalibration is not performed if there is not sufficient data to do so.

**Detailed overview of the invention**

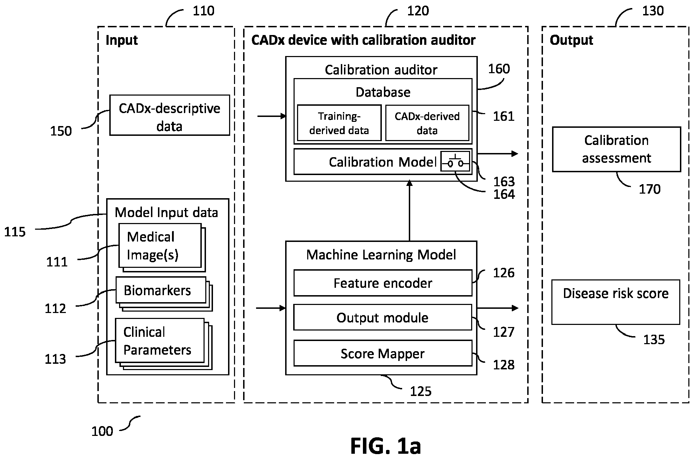

FIG 1a illustrates a CADx device **(120)** with a calibration auditor **(160)** designed to assess if the CADx device is uncalibrated for a particular local population.

The input to the CADx device is a unit of input data **(115),** symbolised by x, containing at least one medical image **(111),** possibly in combination with clinical parameters **(112)** such as patient age and sex, and the result of relevant tests such as biomarkers **(113),** e.g. a test for a gene mutation associated with an increased risk of cancer. Other examples of clinical parameters are results of blood tests or lung function tests, patient or family history of disease, body weight, and location of a suspicious lesion. The input image is one of a CT image, an MRI image, a PET image, an X-ray image, an ultrasound image or a SPECT image. Like standard CADx devices, when the input data **(115)** is provided via the input circuit **(110)** of the CADx device, the data is processed by a machine learning model **(125)** that is trained to predict a risk score of a disease given the input data. Specifically, the machine learning model **(125)** performs a series of mathematical operations on the values of the input data using a feature encoder **(126)** resulting in a set of features, *v*. The output module **(127)** performs a further set of mathematical operations on the features to generate a raw disease risk score, y'. The raw disease risk score is transformed by the score mapper **(128)** into a disease risk score, *y* (135), that is provided via the output circuit **(130).**

Unlike standard CADx devices, the CADx device with calibration auditor **(100)** includes a calibration auditor **(160),** a circuit capable of assessing whether the CADx device is calibrated to any given patient population. Typically, this will be used to determine if the CADx device is calibrated to the specific local patient population served by the clinic (or collection of clinics such as a hospital network) at which the CADx device is being used. The calibration auditor contains a database **(161)** to store the CADx- and training-derived data. CADx-derived data may include both *CADx-input-derived data,* which includes data derived from the inputs to the CADx device, and/or *CADx descriptive data,* which includes data associated with CADx-derived data useful for characterising the expected input data to the CADx device. Correspondingly, training-derived data may include both data *training-input-derived data,* which includes data derived from the machine-learning-model training data and/or *training descriptive data,* which includes associated with the training data. In particular, both CADx and training descriptive data can include local population factors specific to the expected input data to the CADx device at the site at which it is installed and the training data, respectively. CADx-descriptive data **(150)** can be supplied via the input circuit **(110).** The local population factors comprise of data useful for characterising the population of patients for whom input data to the device corresponds, for instance, the prevalence of lung cancer or histoplasmosis, the age distribution, sex, ethnicity and racial distribution of a clinic's patients, the proportion of patients that smoke, the distribution of pack years (i.e. the number of packs of cigarettes a patient smoked per day multiplied by the number of years a patient has smoked) of the clinic's patients, the proportion of the clinic's patients who live in poverty and the seasonal variations in weather such as daytime or night-time temperature and average rainfall. Descriptive data could also include other factors such as imaging factors like the make and model of CT scanner used, the type of imaging protocols used for CT scans, and the typical radiation dose of CT scans at the site. Each factor in the descriptive data may be available separately for patients with different diagnoses, e.g. separately for cancer and benign-disease patients. Furthermore, descriptive data may also contain correlations between each of the descriptive factors. Descriptive data are not mandatory for the CADx device with a calibration auditor but can facilitate both assessing if the CADx device is uncalibrated and the recalibration of the CADx device.

The calibration auditor **(160)** also uses a calibration model **(163)** to measure the difference between the training-derived data, and the CADx-derived data. This difference can be reported to the user in the form of a calibration assessment **(170)**. If the difference between the training-derived data, and the CADx-derived data measured by the calibration model **(163)** is too high, the calibration assessment **(170)** will warn the user that the device is uncalibrated. This warning could be a visual and/or audio warning within the interface of the CADx device that explains to the user that the device is uncalibrated for the local population. The calibration model **(163)** may also include a safety lock **(164)** to prevent the output of a calibration assessment **(170)** if there is not sufficient data to do so. In some embodiments of the invention, the indication of the calibration state may be a visual or audio output that the CADx system is calibrated to the local population.

On the basis of a poor calibration assessment **(170),** the user can elect to discount the disease risk score **(135)** that will be produced from the CADx system.

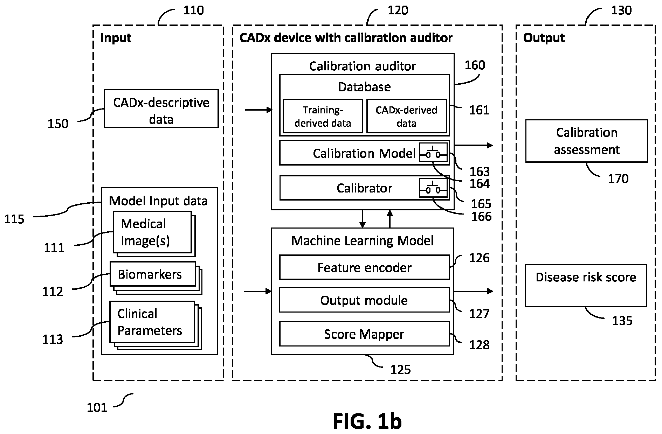

FIG 1b similarly illustrates a CADx device **(120)** with a calibration auditor **(160)** designed to assess if the CADx device is uncalibrated. In addition, this calibration auditor **(160)** is also designed to recalibrate the CADx device, when it has been determined that the CADx device is uncalibrated for the particular local population. Therefore, rather than discounting the disease risk score **(135)** on the basis of a poor calibration assessment **(170),** the user may activate the calibrator **(165)** to update the machine learning model **(125)** to improve the calibration. To do this, the calibrator generates and applies a set of parameter updates for the output module and the score mapper output module to recalibrate the CADx device. The calibrator **(165)** may also include a safety lock *(166)* to prevent recalibration if there is not sufficient data to do so. In this embodiment of the invention, the calibrator is activated by the user, however, in alternate embodiments of the invention, the calibrator may be automatically activated.

**An implementation of the calibration auditor**

FIG 2 shows a diagram of an example embodiment of the calibration auditor **(200).** In this embodiment, the CADx-derived data stored in the database **(161)** of the calibration auditor **(160)** includes the normalised distribution of raw disease risk scores, $\rho_{y'}^{p}$, that is generated from the outputs of the output module taken over a period of time. The training-derived data comprises of a normalised distribution of raw disease risk scores for patients with cancer in the training data, $\rho_{y'}^{c}$, and a normalised distribution of raw disease risk scores for patients with benign disease in the training data, $\rho_{y'}^{b}$. The parameters of the score mapper **(128),** $p_m$, in this embodiment comprise of a single real number between zero and one, $\alpha$, which defines an assumed prevalence of cancer for patients at the site at which the CADx device is installed. From $\alpha$, a distribution of raw disease risk scores,

$$\rho(y'; \alpha) = \alpha\, \rho_{y'}^{b} + (1 - \alpha)\ \rho_{y'}^{c} \qquad (1),$$

is encoded in the score mapper. The score mapper uses the distribution of disease risk scores to calculate which decile a raw disease risk score lies in. The raw disease risk score decile is then provided as a disease risk score **(135)** to the output circuit **(130).**

In this embodiment, the calibration model **(163)** compares $\rho_{y'}^{p}$ to p($y'$; $\alpha$) using a *statistical divergence model* that measures the similarity of two distributions. Here, in a preferred embodiment of the invention, the calibration model uses the Kullbach-Liebler Divergence (KLD), which is given by the following equation:

$$D_{KL} = \sum_{y' \epsilon Y} \rho(y'; \alpha) \log \frac{\rho(y'; \alpha)}{\rho_{y'}^{p}(y')} \qquad (2)$$

The KLD is provided as a calibration assessment **(170)** to the user via the output circuit. When value of the KLD are above a threshold, which is set at training time or at installation time, for instance, one, the user will know that the CADx device is mis-calibrated. In some cases the CADx device may be recalibrated, after the determination that the device is uncalibrated. A threshold for the KLD may optionally be stored within calibration model **(263).** When the KLD is above the KLD threshold, indicating that the CADx device is uncalibrated, the calibration assessment **(170)** indicates to the user that the CADx device is uncalibrated.

In some examples of the invention, an appropriate value for the KLD threshold may be calculated from the training-derived data, for example by calculating the KLD between $\rho(y'; \alpha)$ and $\rho(y'; \alpha + 0.05)$, where the assumed

prevalence of cancer, $\alpha$, can be obtained from the literature or simply set to a standard value such as 0.3.

Many other standard statistical divergence models are available that could be used instead of the KLD, such as the Kolmogorov-Smirnov statistic, the Anderson-Darling statistics, or the Kuiper's statistic. For instance, if the Kolmogorov-Smirnov statistic was used:

$$D_{KS} = \sup_{y'} |F_\alpha(y') - F^p(y')| \qquad (3)$$

where $F_\alpha$ and $F^p$ are the empirical cumulative distribution functions for $\rho(y'; \alpha)$ and $\rho^p_{y'}(y')$ respectively, and the distributions are represented as empirical distribution functions, and the threshold if defined by the ability to reject the null hypothesis that the two distributions are the same at given level, e.g. for a level of 5%, the calibration would indicate "uncalibrated" if the

$$D_{KS} > 1.358 \sqrt{\frac{N_t}{N_p}} \qquad (4)$$

where $N_t$ and $N_p$ are the sample sizes for $F_\alpha$ and $F^p$, respectively.

**Detecting when sufficient CADx-derived data exists to provide a calibration assessment**

To ensure that a calibration assessment **(170)** is not provided without sufficient data available to do so, a safety-lock circuit **(264)** may be included in the calibration model **(263)**. A simple implementation of this safety-lock circuit could prevent a calibration assessment **(170)** from being provided if the number of datasets in the CADx-derived data in the database **(261)** is less than a set number, e.g. 100. Another implementation could prevent a calibration assessment **(170)** from being provided if there are less than at least a set number of datasets per some strata of data within the database, e.g. 10 datasets within each raw disease risk score range of [0;0.1), [0.1;0.2), ... [0.9;1.0] that have received a CADx score.

In some examples of the invention, a more general implementation of the safety-lock circuit **(164)** would be to use a statistical method to calculate the uncertainty in the calibration assessment **(170),** and prevent it being provided if the uncertainty is too high. For instance, bootstrap methods could be used to bootstrap the data within the database **(261)** and use this bootstrapped data to calculate a confidence interval in the output of the calibration model **(163)**, e.g. the KLD. The safety-lock circuit **(164)** could then prevent the calibration assessment **(170)** from being provided if the confidence interval was too large. An example of a method to determine if the KLD confidence interval is too large would be if it contained KLD threshold, indicating that it was uncertain whether the CADx device was uncalibrated or not. Another method could be to choose a reasonable threshold above which the confidence interval width should not be above.

If necessary, CADx-derived data from other sites at which the device is installed and which have an equivalent patient population can be input to the CADx device to augment the CADx-derived data such that there is enough to provide a calibration assessment.

**Implementation of a calibrator**

In some embodiments of the invention, the CADx device may also be equipped with a calibrator **(165),** which the user may activate when they have identified that the CADx device is not calibrated.

The calibrator may be implemented in many ways. In this embodiment, the calibrator **(165)** may comprise of a circuit that optimises the parameters of the score mapper **(228)** such that the similarity between the distributions $\rho(y'; \alpha)$ and $\rho^p_{y'}(y')$ is maximized, which in turn improves the calibration. In this embodiment, the similarity between the distributions is measured by the KLD statistical divergence model. This similarity is maximized by minimizing the KLD with respect to the score mapper parameter $\alpha$. This could be accomplished by varying the score mapper parameter $\alpha$ (the cancer prevalence) over a range of values for instance, the values 0, 0.01, 0.02, ..., 1. The calibrator then selects the value of $\alpha$ that gives the highest KLD:

$$\alpha \leftarrow argmax_\alpha D_{KL} \qquad (5)$$

The new value for $\alpha$ is stored in the score mapper, replacing the previous value.

An identical method could be used if the calibration model **(163)** used a different statistical divergence model.

**Detecting when sufficient CADx-derived data exists to activate the calibrator**

Similar to the calibration model **(163)**, a safety-lock circuit **(166)** may be included in the calibrator **(165)** to prevent recalibration of the CADx device **(120)** without sufficient data available to do so. Many of the implementations of the calibration-model safety-lock circuit **(164)** are also suitable for the calibrator safety-lock circuit **(166)**. Such as preventing recalibration if the number of datasets in the CADx-derived data in the database **(161)** is less than a set number, e.g. 100, or if there are less than a set number of datasets, e.g. 10, within each raw disease risk score range of [0;0.1), [0.1;0.2), ... [0.9;1.0] have received a CADx score.

Like the calibration model **(163)**, a more general implementation of the safety-lock circuit **(166)** in the calibrator **(165)** would be to use a statistical method to calculate the uncertainty in the recalibration and prevent the recalibration from being applied to the CADx device if the uncertainty is too high. For instance, bootstrap methods could be used to bootstrap the data within the database **(161)** and use this bootstrapped data to run the calibrator **(165)** many times. The uncertainty in recalibration could then be assessed by using the results of running the calibrator **(165)** on the bootstrapped data to calculate a confidence interval in the proposed parameter updates to the score mapper and/or output module. In the embodiment shown in FIG 2, this would be a confidence interval in the new value for $\alpha$ proposed by the calibrator. The safety-lock circuit **(164)** could then prevent parameters from being updated if the confidence interval was too large. An example of a method to determine if the $\alpha$ confidence interval is too large would be simply to choose a reasonable threshold the confidence internal width should not be above e.g. 0.2.

Another way to assess the uncertainty in the recalibration would be to use the results of running the calibrator **(165)** on the bootstrapped data to calculate a confidence interval in the assessed calibration of the recalibrated models, i.e. in the output of the calibration model **(163)** such as the KLD. An example of a method to determine if the KLD confidence interval across the recalibrated models is too large would be if it contained KLD threshold, indicating that it was uncertain whether the CADx device was uncalibrated or not. Another method could be to choose a reasonable threshold above which the confidence interval width should not be above. In some embodiments of the invention, the threshold is a predetermined threshold that may be set either during training of the machine learning model, or at the time of installation of the CADx system.

If necessary, CADx-derived data from other sites at which the device is installed and which have an equivalent patient population can be input to the CADx device to augment the CADx-derived data to enable an earlier calibration.

**Alternative embodiment of the calibration auditor**

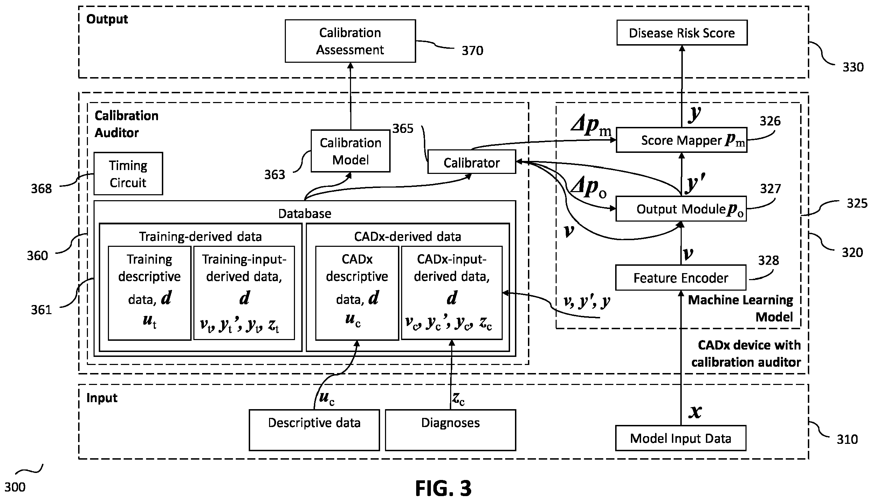

An alternative embodiment of the CADx device with a calibration auditor **(320)** is shown in FIG 3. In this embodiment, CADx-derived data comprises of CADx-input-derived data, which includes the features, $v_c$, raw disease risk scores, $y_c'$, and disease risk scores, $y_c$, derived from inputs to the machine learning model **(125)** from usage of the CADx device at a given site or collection of sites. Optionally, corresponding patient diagnoses, $z_c$, can also be supplied as input to the CADx device to be stored in the database **(361)**. CADx descriptive data, $u_c$, may also be supplied as input to the CADx device and stored in the database **(361)**.

Similarly, the training-derived data comprises of training descriptive data, $u_t$, and training-input-derived data, which includes the features, $v_t$, raw disease risk scores, $y_t'$, disease risk scores, $y_t$, and diagnoses, $zt$, corresponding to the training data.

Given this database, there are many ways in which the calibration model **(363)** could measure the difference between the training- and CADx-derived data to identify if the CADx device was uncalibrated. This difference measure may involve one or more of the CADx/training-derived input data, the CADx/training descriptive data, and the relationship between the two.

In some examples of the invention, the calibration model **(363)** will use the disease risk scores, $y_c$, and diagnoses, $z_c$, to measure the number of false positives i.e. patients with negative cancer diagnoses that receive disease risk scores above a threshold that indicates cancer is likely and/or false negatives i.e. patients with positive cancer diagnoses that receive disease risk scores below a threshold that indicates cancer is unlikely. If the frequency of such false positives and/or false negatives over a period of time is above a certain threshold e.g. substantially higher than that recorded during training, says 20% higher, the calibration model **(363)** indicates to the user that the device is uncalibrated.

In some examples of the invention, the calibration model **(363)** could use the distribution of machine-learning-model features to measure this difference. A possible implementation could be as follows: First, the calibration model **(363)** calculates the distribution of features in the training data from the features stored in the training-input-derived data, $\rho(v_t)$, and the distribution of features in the CADx data from stored in the CADx-input-derived data, $\rho(v_c)$. To produce the calibration assessment **(170)**, the calibration model **(363)** computes the similarity between the distributions $\rho(v_t)$ and

$\rho(\boldsymbol{v_c})$ using a statistical divergence model, such as the KLD. This similarity may be used to generate a calibration assessment **(170)** in an equivalent manner to the embodiment shown in FIG 2. If patient diagnoses have been supplied by the user, the calibration model may also take these into account. For instance, the above computation of similarity based on feature distributions could be done separately for features from cancer patients and benign-disease patients.

In some examples of the invention, the calibration model **(363)** could use the CADx/training descriptive data to produce the calibration assessment **(170)**. This has the advantage that CADx descriptive data may be available before the CADx device has been used, allowing the calibration to be assessed as early as at the point of installation before CADx-input derived data is available. There are many statistical tests to determine the difference between two populations based on descriptive factors that could be used in this implementation. For example, for each factor that appears in the descriptive data, a univariate t-test could be used to determine if there is a significant difference between the CADx and training data. If a significant difference was found, the calibration model could produce a calibration assessment that reported the CADx device was uncalibrated. Alternative implementations could use a multivariate statistical test, such as the Hotelling test or multivariate analysis of variance. If the descriptive data are given separately for patient populations with differing diagnoses, the calibration model may also take these into account. For instance, the above statistical tests could be done separately for descriptive data corresponding to cancer patients and benign-disease patients.

These two implementations could also be used in combination to take account of both the CADx/training-derived input data and the CADx/training descriptive data. In another implementation that uses both input and descriptive data to assess calibration, the database **(361)** could include correlations between descriptive and input data. These correlations can then be used in its calculation of the difference between the CADx- and training-derived data. For example, consider the correlation matrix between machine-learning-model features and descriptive factors for the training-derived data, $S(\boldsymbol{v_t}, \boldsymbol{u_t})$, and the CADx-derived data, $S(\boldsymbol{v_c}, \boldsymbol{u_c})$. To produce a calibration assessment **(170)**, the calibration model **(363)** can compare these two sets of correlations by the same methods as described for comparing descriptive factors described above.

## Alternative embodiment of the calibrator

In the embodiment depicted in FIG 3, the CADx- and training-derived data available in the database **(361)** as described above, allow for many possible implementations of the calibrator **(365)**. These implementations may retrain both the score mapper **(126)** parameters, $p_m$, and/or the output module **(127)** parameters, $\boldsymbol{p_o}$, by retraining (or fine tuning) the score mapper **(126)** and/or output module **(127)** using the CADx-derived data and training-input-derived data.

An example of a method to recalibrate the CADx device by retraining the score mapper **(126)** would be for the calibrator **(365)** to use an optimisation technique such as isotonic regression to retrain the score mapper parameters using the raw disease prediction scores, $\boldsymbol{y_c}'$, and diagnoses, $\boldsymbol{z_c}$, from the database **(361)**,

An example of a method to recalibrate the CADx device by retraining the output module **(127)** would be for the calibrator **(365)** to use the features, $\boldsymbol{v}$, and diagnoses, $\boldsymbol{z}$, from the database **(361)** to retrain the output module. This method may use features and diagnoses from either the training-input-derived data, the CADx-input-derived data, or a combination of both.

As shown in the process **(500)** shown in FIG 5, in some examples of the invention, the training of the output module **(127)** may entail using a sampler **(510)** to repeatedly sample at least one set of features, $\boldsymbol{v}$, from the database **(161)** to the output module **(127)** to obtain raw disease prediction scores, $\boldsymbol{y}'$. In some examples of the invention, the difference between the raw disease prediction score, $\boldsymbol{y}'$ and the ground-truth diagnosis), z, may be computed using a loss function **(520)** which computes a loss value **(525)** which is chosen to measure how accurately the model predicts the labels given the associated data. In some examples of the invention, an optimiser **(530)** running an optimization may be used to reduce the loss **(525)** by measuring how much each of the output-module parameters, $\boldsymbol{p_o}$, contributed to the loss and using the information to calculate and apply output-module parameter updates, $\varDelta\boldsymbol{p_o}$, in such a way as to reduce the loss **(525)**. Each such modification is referred to as an iteration **(540)**. After enough iterations **(540)**, the output module **(127)** will have been recalibrated.

Examples of possible loss functions **(520)** include the squared error, the cross-entropy loss, hinge loss, exponential loss, and tangent loss.

Examples of possible optimizers **(530)** include the Broyden-Fletcher-Goldfarb-Shanno algorithm (BFGS), the Newton-Raphson method, the Nelder-Mead method, the conjugate gradient method, stochastic gradient decent (SGD) with or without momentum, adaptive moment estimation (Adam), root mean square prop (RMSProp), adaptive delta (Adadelta), or adaptive gradient (Adagrad).

In some examples of the invention, the number of iterations **(540)** will be a fixed number, such as 100 iterations. In other examples of the invention, retraining iterations **(540)** will continue to be applied until one or more conditions are met, for example, until the parameter updates for the output module **(127)** are below a certain threshold, e.g. $|\varDelta p_o| < 10^{-4}$.

In some examples of the invention, if diagnoses, $\boldsymbol{z_c}$, are available in the CADx-input-derived data, the output module **(127)** can be retrained by sampling CADx-input-derived features, $\boldsymbol{v_c}$, and diagnoses, $\boldsymbol{z_c}$, from the database

(361). In some examples of the invention, the output module (127) can be retrained by sampling a combination of both CADx- and training-input-derived features, $v_c$ and vt, and diagnoses, $z_c$ and $z_t$, from the database (361).

**Alternative embodiment of calibrator where diagnoses may not be available**

There are many ways to calibrate the CADx device using both the CADx- and training-input-derived data and the CADx- and training-descriptive data, if diagnoses, $z_c$, are not available in the CADx-input-derived data,

In some examples of the invention, the lack of diagnoses, $z_c$, in the CADx-input-derived data, can made up for by sampling or generating data to recalibrate the CADx device from the training-derived data. For example, the score mapper (126) and/or output module (127) can be retrained by sampling training-input-derived features, vt, and diagnoses, zt, from the database (361), using the sampler (510) to select data for retraining such that the distribution of features sampled, $\rho(v_t)$, matches that of the distribution of features in the CADx-input-derived data, $\rho(v_c)$.

In some examples of the invention, CADx and training descriptive data can also be used by the sampler (510) to inform the sampling strategy. For example, correlations between the descriptive data and machine-learning-model features could be used to sample or generate data from the training-derived data. In some examples of the invention, the sampler may use the correlation matrix between descriptive factors and machine-learning-model features, $S(u, v)$, raw disease prediction scores, $S(u,y')$, disease risk scores, $S(u, y)$, and diagnoses $S(u, z)$, for both the CADx- and training-derived data to sample or synthesize data for retraining the score mapper (126) and/or output module (127). For example, a set of CADx descriptive factors, $u_c$, maybe be used in combination with the correlations calculated from the training data, $S(v_t, u_t)$, $S(v_t, y_t')$, $S(v_t, y_t)$, and/or $S(v_t, z_t')$, to infer the expected distributions of features, raw disease prediction scores, disease risk scores, and/or diagnoses in the patient population of the clinic. These inferred distributions can then be used by the sampler to sample or generate data from the training to recalibrate the CADx device. This has the advantage that CADx descriptive data may be available before the CADx device has been used, allowing the CADx device to be recalibrated as early as at the point of installation, before any CADx-input derived data is available.

**Alternative embodiment of the invention where descriptive factors are time dependent**

In some embodiments of the invention, one or more of the CADx-descriptive factors # factors may be the change in the prevalence of a disease such as histoplasmosis in each month of the year, or each quarter of the year. In this embodiment, the date that is associated with the input data is supplied as an input to the CADx device and the calibrator holds one set of mapping parameters for each month of the year. Input data typically has an associated timestamp, but if not, the date can be obtained from a timing circuit (368). The calibrator updates the parameters of the score mapper with the set of parameters appropriate to the date associated with the input data. Other factors known to affect local population factors could also be used, for instance daily temperatures or weekly rainfall.

**Alternative embodiment of the invention where input data is stored**

In some embodiments of the invention, the CADx and/or training input data, $x_c$ and $x_t$, are stored in the database (361). When features, $v$, are required by the calibration model (363) or the calibrator (365), they can be computed by inputting the input data input the feature encoder (128) of the machine learning model (125).

N10. The devices in N1-9 where the input database stores information on patient biopsies, diagnoses, and which patients have been discharged or referred, to enable the calibration model to detect if the CADx device is uncalibrated and to obtain a more accurate calibration.

When deploying a CADx device, it is critical to have mechanisms for detecting when the device is not well calibrated to the local patient population. Due to the difficulty in detecting that a CADx device is uncalibrated, no commercial CADx devices (that the inventors know of) offer this capability. The advantage of the device is it allows the users to identify when the CADx device is uncalibrated to their local population allowing them to better decide when to ignore the disease risk score reducing the risk of unnecessary biopsies or delayed diagnoses.

Moreover, even when the CADx device is known to be uncalibrated to a local population, there may not be enough data available to recalibrate the device. Hence the invention has the advantage of identifying when there is insufficient data available and blocking any update to the machine learning model parameters that would make the device hazardous to use. In addition, the device can offer an estimate of how much longer data needs to be accrued before the device can be recalibrated. Finally, the device provides methods to update the internal parameters of the machine learning model to provide a better calibration than is possible with state-of-the-art CADx devices.

This invention can be applied in any context where a CADx device is used to assess the risk of disease from input medical data such as medical images.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for receiving at least one input medical image of a patient in which the patient's lungs are visible.

The computer program may be stored internally on a tangible and non-transitory computer readable storage medium or transmitted to the computer system via a computer readable transmission medium. All or some of the computer program may be provided on computer readable media permanently, removably or remotely coupled to an information processing system. The tangible and non-transitory computer readable media may include, for example and without limitation, any number of the following: magnetic storage media including disk and tape storage media; optical storage media such as compact disk media e.g., CD ROM, CD R, etc. and digital video disk storage media; non-volatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing running program or portion of a program, current program values and state information, and the resources used by the operating system to manage the execution of the process. An operating system OS is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output I/O devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

**Claims**

1. A CADx system for analysing at least one input medical image from a specified local population for a disease risk score and determining if the system is calibrated for the local population comprising:

   an input circuit for receiving at least one input medical image and producing CADx derived data based on the received input image;
   a machine learning model for determining a disease risk score for the input medical image;
   a calibration auditor circuit for determining a calibration state of the CADx system by: receiving the CADx derived data and comparing the CADx derived data to training derived data, where the training derived data comprises one of more of: training data for the machine learning model for determining the disease risk score, and data on local population factors associated with the training data; and
   an output circuit for outputting a disease risk score for the input medical image and an indication of the calibration state for the CADx system.

2. A CADx system according to claim 1 wherein the calibration auditor circuit further comprises a safety lock that prevents outputting of the indication of the calibration state when there is insufficient CADx derived data to determine the calibration state.

3. A CADx system according to claim 2 wherein the calibration auditor circuit further comprises a calibrator to recalibrate the CADx system when the calibration state is determined to be uncalibrated for the specified local population.

4. A CADx system according to claim 1 wherein the calibration auditor circuit further comprises a threshold for calibration state determination, wherein, when the difference obtained by comparing the CADx derived data and the training derived data, exceeds the threshold, the CADx system indicates that it is uncalibrated .

5. A CADx system according to claim 5 wherein the threshold is a predetermined threshold that is set either during training of the machine learning model or at the time of installation of the CADx system.

6. A CADx system according to claim 5 wherein the CADx system is recalibrated after the determination that the system is uncalibrated.

7. A CADx system according to claim 1 wherein the input medical image is one of: a CT image, an MRI image, a PET image, an X-ray image, an ultrasound image or a SPECT image and the input further comprises one or more of: biomarkers for the patient or clinical parameters for the patient; wherein the biomarkers and clinical parameters comprise at least one of: patient age, patient sex, results of blood tests, results of lung function tests .

8. A method of determining that a CADx system is uncalibrated for a particular local population, comprising the steps of:

   providing an input to the CADx system, comprising at least one medical image from the particular local population, to a machine learning model to provide at least one CADx derived data;
   providing the at least one CADx derived data from the machine learning model to a calibration auditor, where the calibration auditor compares the CADx derived data to calibration training data,
   wherein the calibration training data comprises one of more of: training data for the machine learning model, data on local population factors associated with the training data; and
   when the difference between the CADx derived data and the calibration training data exceeds a predetermined threshold, the CADx system is determined to be uncalibrated for the particular local population.

9. A method as claimed in claim 10 wherein the CADx derived data is one or more of data derived from the CADx input, intermediate data from the machine learning model or outputs from the CADx system.

10. A method according to claim 10 wherein the CADx-derived data includes the local population factors; wherein the local population factors comprise one of more of age distribution, sex distribution, race or ethnicity distribution for the local population, seasonal variations in weather for the area of the local population; parameters related to the image scanner for the at least one medical image input.

11. A method according to claim 10, wherein, when the CADx system is determined to be uncalibrated, the CADx system is recalibrated for the local population by the following steps:

   activating a calibrator within the calibration auditor;
   using the activated calibrator to update the machine learning model;

12. A method as claimed in claim 16 wherein if the CADx system determines that there is not sufficient data in the calibration auditor database, a safety lock prevents the CADx system from being recalibrated.

13. A method according to claim 13 wherein the machine learning model comprises at least an output module and a score mapper, and the calibrator updates the machine learning model by retraining at least one of the output module and score mapper using at least one of features $v_c$ and diagnoses $z_c$ from the CADx-derived data and at least one of features vt and diagnoses zt from the training-derived data.

14. A method according to claim 10 wherein the calibration auditor further comprises a threshold for recalibration, wherein, when a comparison of the the CADx derived data with the training derived data, exceeds the threshold, the CADx system is recalibrated .

15. A method according to claim 19 wherein the comparison of the CADx derived data and the training derived data is done with a statistical divergence model that is one of more of: a Kullbach-Liebler Divergence (KLD) where $D_{KL} =$

$$\sum_{y' \epsilon Y} \rho(y'; \alpha) \log \frac{\rho(y'; \alpha)}{\rho^p_{y'}(y')}$$

, where p(y'; $\alpha$) is calculated from the training data dt, and p$^p_{y'}$ is calculated from the CADx derived data, or Kolmogorov-Smirnov statistic, the Anderson-Darling test, or Kuiper's test.

**FIG. 1a**

**FIG. 1b**

FIG. 2

**FIG. 3**

EP 4 231 313 A1

FIG.4

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 686 805 A1 (KONINKLIJKE PHILIPS NV [NL]) 29 July 2020 (2020-07-29) * The whole document, in particular: Paragraphs [0014], [0021], [0024], [0026], [0037], [0043] – [0045], [0051], [0066] – [0069], [0075] – [0079], [0080], [0084] – [0087]; Figure 3a. * ----- | 1–15 | INV. G16H50/20 G06N7/01 G06N20/00 G16H30/40 G16H40/40 G16H50/30 G16H50/70 |
| X | EIKE PETERSEN ET AL: "Responsible and Regulatory Conform Machine Learning for Medicine: A Survey of Challenges and Solutions", ARXIV.ORG, CORNELL UNIVERSITY LIBRARY, 201 OLIN LIBRARY CORNELL UNIVERSITY ITHACA, NY 14853, 20 July 2021 (2021-07-20), XP091243023, DOI: 10.1109/ACCESS.2022.3178382 * The whole document, in particular: Abstract; Figure 1. * ----- | 1–15 | |

TECHNICAL FIELDS SEARCHED (IPC)

G16H
G06N

The present search report has been drawn up for all claims

| Place of search | Date of completion of the search | Examiner |
|---|---|---|
| Munich | 12 July 2023 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 6600

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2023

| Patent document cited in search report | | Publication date | Patent family member(s) | | Publication date |
|---|---|---|---|---|---|
| EP 3686805 | A1 | 29-07-2020 | CN | 113366499 A | 07-09-2021 |
| | | | EP | 3686805 A1 | 29-07-2020 |
| | | | EP | 3918529 A1 | 08-12-2021 |
| | | | JP | 2022518286 A | 14-03-2022 |
| | | | US | 2022083814 A1 | 17-03-2022 |
| | | | WO | 2020156924 A1 | 06-08-2020 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PLATT, J.** Probabilistic outputs for support vector machines and comparisons to regularized likelihood methods. *Advances in Large Margin Classifiers,* 1999, vol. 10 (3), 61-74

- **NICULESCU-MIZIL A ; CARUANA R.** Predicting good probabilities with supervised learning. *ICML '05: Proceedings of the 22nd international conference on Machine learning,* August 2005, 625-632, https://doi.org/10.1145/1102351.1102430
- **MIRSAEIDI M et al.** Racial Difference in Sarcoidosis Mortality in the United States. *Chest,* February 2015, vol. 147 (2), 438-449